# EUROPEAN PATENT APPLICATION

(11) **EP 4 272 669 A1**
(43) Date of publication of application: **08.11.2023**
(21) Application number: 21912470.8
(22) Date of filing: 20.01.2021
(51) Int. Cl.: A61B 18/12

(54) **RADIO FREQUENCY OPERATION SAFETY CONTROL METHOD, DEVICE AND RADIO FREQUENCY HOST**

(30) Priority: 31.12.2020 CN 202011642360
(71) Applicant: Hangzhou Broncus Medical Co., Ltd., Hangzhou, Zhejiang 310051 (CN)
(72) Inventor: XU, Hong, Hangzhou, Zhejiang 310051 (CN); CUI, Changjie, Hangzhou, Zhejiang 310051 (CN); LIU, Zhiyu, Hangzhou, Zhejiang 310051 (CN)
(74) Representative: Petraz, Gilberto Luigi
(86) International application number: PCT/CN2021/072957
(87) International publication number: WO 2022/141690

(57) **Abstract**

Disclosed are a method and device for safety control of radio frequency operation, and a radio frequency host. The method for safety control of radio frequency operation includes: when connecting ends of a plurality of radio frequency circuits connect an operated object to a radio frequency host, acquiring detection values of the plurality of radio frequency circuits; determining whether change amounts of the detection values reach a preset value range; when a number of target radio frequency circuits reaching the preset value range is not less than a preset number, selecting the preset number of target radio frequency circuits from the target radio frequency circuits according to a preset selection rule as radio frequency input circuits, and inputting radio frequency energy into the radio frequency input circuits; when the number of target radio frequency circuits reaching the preset value range is less than the preset number, not inputting radio frequency energy into any radio frequency circuit. By automatically detecting connection quality between the radio frequency host and the operated object, safety and intelligence of radio frequency operations are improved.

## Description

### TECHNICAL FIELD

Embodiments of this application relate to the field of electronic technologies, and in particular to a method and device for safety control of radio frequency operation, and a radio frequency host.

### BACKGROUND

A neutral electrode is used in conjunction with a high-frequency host such as a radio frequency instrument, and is connected to the high-frequency host to provide a low-density return channel for the high-frequency host. It is usually composed of conductive glue, aluminum foil, foam, anti-allergic layer, etc. A degree of fit between a neutral electrode and an operation target may affect an operation process. If a connection for a neutral electrode is in a poor contact, it may cause damage to a device or harm to a user during an operation process such as performing ablation using a high-frequency host device.

In the prior art, an alarm prompt is usually given when it is judged that a neutral electrode is abnormally attached. If an operator is not aware of this mechanism for some reason, it will cause operational risks to an operation target, or cause damage to a high-frequency host device. This prompt method is not sufficiently intelligent.

### SUMMARY

### Technical problem

Embodiments of this application provides a method and device for safety control of radio frequency operation, and a radio frequency host, which can improve safety and intelligence of radio frequency operations when connection between a radio frequency host and a radio frequency operated object does not meet a standard.

### Technical Solution

An aspect of embodiments of this application provides a method for safety control of radio frequency operation, including steps of:
when connecting ends of a plurality of radio frequency circuits connect an operated object to a radio frequency host, acquiring detection values of the plurality of radio frequency circuits; determining whether change amounts of the detection values reach a preset value range; when a number of target radio frequency circuits of which the change amounts of the detection values reach the preset value range is not less than a preset number, selecting the preset number of target radio frequency circuits from the target radio frequency circuits according to a preset selection rule as radio frequency input circuits, and inputting radio frequency energy into the radio frequency input circuits; when the number of target radio frequency circuits of which the change amounts of the detection values reach the preset value range is less than the preset number, not inputting radio frequency energy into any radio frequency circuit.

An aspect of embodiments of this application further provides a device for safety control of radio frequency operation, including:
an acquiring module configured to acquire detection values of a plurality of radio frequency circuits when connecting ends of the plurality of radio frequency circuits connect an operated object to a radio frequency host;
a determining module configured to determine whether change amounts of the detection values reach a preset value range; and
a processing module configured to: select a preset number of target radio frequency circuits from a plurality of target radio frequency circuits according to a preset selection rule as radio frequency input circuits, and input radio frequency energy into the radio frequency input circuits when a number of target radio frequency circuits of which the change amounts of the detection values reach the preset value range is not less than the preset number;
wherein the processing module is further configured not to input radio frequency energy into any radio frequency circuit when the number of target radio frequency circuits of which the change amounts of the detection values reach the preset value range is less than the preset number.

An aspect of embodiments of this application further provides a radio frequency host, including:
a memory and a processor, wherein the memory stores executable program codes; the processor coupled with the memory calls the executable program codes stored in the memory to execute the aforesaid method for safety control of radio frequency operation.

### Advantageous effect

From the above embodiments of this application, it can be known that: when an operated object is connected to a radio frequency host through connecting ends of radio frequency circuits, according change amounts of detection values of the radio frequency circuits, it is determined whether a number of target radio frequency circuits of which the change amounts reach a preset value range reaches a preset number, that is, it is determined whether the connection between the connecting ends and the operated object meets a connection standard; if reaching, the preset number of target radio frequency circuits are selected from the target radio frequency circuits as radio frequency input circuits, and radio frequency signals are controlled to input; if not reaching, no radio frequency is input, so as to avoid subsequent radio frequency operations from being affected by connection not meeting the standard. Accordingly, the above-described method for safety control of radio frequency operation can automatically determine whether connection between an operated object and radio frequency circuits meets a standard, and does not input radio frequency energy when the number of radio frequency circuits meeting the standard do not meet requirement of radio frequency operation, thereby improving safety and intelligence of radio frequency operation.

### BRIEF DESCRIPTION OF THE DRAWINGS

In order to describe the technical solutions according to the embodiments of the present invention or in the prior art more clearly, the drawings needed to be used in the embodiments or in the prior art will be described briefly below. Apparently, the drawings in the following description show some embodiments of the present application. Other drawings can be obtained by persons of ordinary skill in the art based on these drawings without creative efforts.
FIG. 1 is a schematic diagram of an application scene of a method for safety control of radio frequency operation provided by an embodiment of this application.
FIG. 2 is a schematic flow chart of a method for safety control of radio frequency operation provided by an embodiment of this application.
FIG. 3 is a schematic flow chart of a method for safety control of radio frequency operation provided by another embodiment of this application.
FIG. 4 is a structural schematic diagram of a radio frequency circuit in a method for safety control of radio frequency operation provided by an embodiment of this application.
FIG. 5 is a schematic flow chart of a method for safety control of radio frequency operation provided by another embodiment of this application.
FIG. 6 is a structural schematic diagram of an impedance detection circuit in a method for safety control of radio frequency operation provided by an embodiment of this application.
FIG. 7 is a structural schematic diagram of a device for safety control of radio frequency operation provided by an embodiment of this application.
FIG. 8 is a structural schematic diagram of a radio frequency host provided by an embodiment of this application.

### DESCRIPTION OF THE EMBODIMENTS

In order to make the objects, technical solutions and advantages of the embodiments of the present invention clearer, the technical solutions according to the embodiments of the present invention will be clearly and completely described with reference to drawings in the embodiments of the present invention. Apparently, the embodiments described are merely some embodiments, but not all of the embodiments of the present application. All other embodiments obtained by ordinary persons skilled in the art based on the embodiments of the present invention without creative efforts shall fall within the protection scope of the present invention.

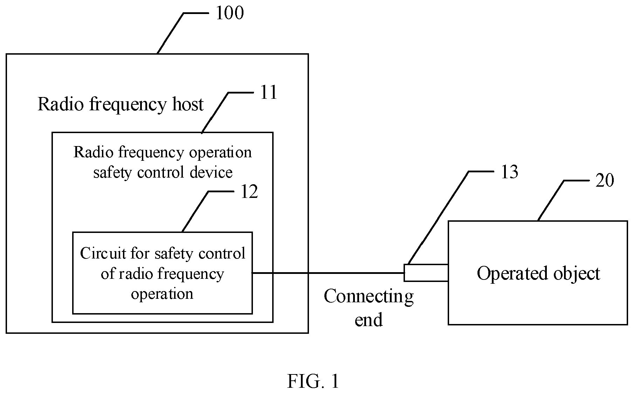

Referring to FIG. 1, which is a schematic diagram of an application scene of a method for safety control of radio frequency operation provided by an embodiment of this application; the method for safety control of radio frequency operation can be used to control safety problems relating to connection between a radio frequency host and a radio frequency operated object through the radio frequency host, and thereby improving safety and intelligence of radio frequency operations.

Specifically, the radio frequency host may be a device such as a radio frequency ablation device, and the radio frequency operated object may be any object that needs a radio frequency operation. For example, when the radio frequency host is a radio frequency ablation device, the radio frequency operated object may be an animal body that needs to ablate mutated tissues in the body. As shown in FIG. 1, a radio frequency host 100 is connected with an operation object 200. The radio frequency host 100 is provided therein with a radio frequency operation safety control device 11 and a radio frequency circuit 12. The radio frequency circuit 12 is used to detect whether a connection between the radio frequency host 100 and the operated object 200 meets a standard, and the radio frequency circuit 12 has a connecting end 13 (specifically, it may be a neutral electrode), the radio frequency host 100 is connected with the operated object 200 through the connecting end 13. The radio frequency host 100 controls whether to output a radio frequency signal and the output power of the output radio frequency signal through the radio frequency operation safety control device 11.

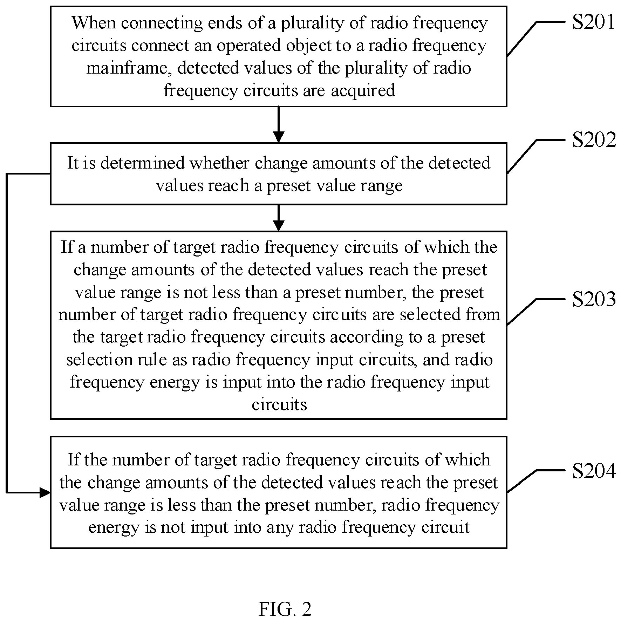

Referring to FIG.2, which is a schematic flow chart of a method for safety control of radio frequency operation provided by an embodiment of this application. The method can be applied to the radio frequency host shown in FIG. 1, as shown in FIG. 2, the method specifically comprises the steps as follows.

Step S201, when the connecting ends of a plurality of radio frequency circuits connects an operated object to a radio frequency host, detection values of the plurality of radio frequency circuits are acquired.

Specifically, an execution subject of this embodiment is the radio frequency host, and the radio frequency host can detect whether a connecting end has been connected to the operation object. The connecting end is used to connect an operated object to the radio frequency host, and whether firmness of the connection meets a connection standard is a prerequisite of whether the radio frequency host can complete a radio frequency operation smoothly and safely.

The connecting end can be a neutral electrode; the operated object is an object or target for the radio frequency host to perform a radio frequency operation.

The radio frequency circuit includes a detection circuit, which is used to detect whether a connection between a connecting end and an operated object meets a connection standard; when the connecting end is a neutral electrode, the detection circuit specifically detects whether an attachment degree between the neutral electrode and the operated object meets an attachment standard.

The radio frequency circuit further includes a radio frequency radio module; under control of the radio frequency host, the radio frequency module inputs a radio frequency signal into the radio frequency circuit to execute a radio frequency operation for the operated object.

Step S202, it is determined whether change amounts of the detection values reach a preset value range.

Whether a change amount of a detection value reaches a preset value range is a determination standard for the radio frequency host to determine whether a connection between a connecting end and an operated object meets a connection standard.

Specifically, the detection value may be an impedance value, and may also be a change value of a voltage value of a primary coil of a transformer.

If a change amount of the detection values reaches a preset value range, the radio frequency host determines that the connection between the connecting end and the operated object meets the connection standard; if the change amount of the detection values does not reach the preset value range, the radio frequency host determines that the connection between the connecting end and the operated object does not meet the connection standard.

Step S203, if a number of target radio frequency circuits of which the change amounts of the detection values reach the preset value range is not less than a preset number, the preset number of target radio frequency circuits are selected from the target radio frequency circuits according to a preset selection rule as radio frequency input circuits, and radio frequency energy is input into the radio frequency input circuits.

For example, if the preset number is 2, that is, when target radio frequency circuits of which connections between connecting ends and the operated object meet the connection standard reach 2, 2 of the target radio frequency circuits are selected from these target radio frequency circuits according to a preset selection rule as radio frequency input circuits, and radio frequency energy is input thereto to be provided to the operated object.

It should be noted that each radio frequency circuit and its connecting end have their own preset series numbers. According to these series numbers, the radio frequency host can know to which operation area of the radio frequency operation the connecting end of the radio frequency circuit is connected, and determine how many radio frequency circuits each operation area needs to connect, that is, how many connecting ends are connected, according to nature of the current radio frequency operation.

The selection rule is: according to an operation area and the number of connecting ends required by the current radio frequency operation, to select a preset number of target radio frequency circuits from the target radio frequency circuits meeting the connection standard as the radio frequency input circuits.

Distribution of the target radio frequency circuits should be capable of meeting selection requirement, and both the operation area and the number of the distribution are met.

Step S204, if the number of target radio frequency circuits of which the change amounts of the detection values reach the preset value range is less than the preset number, radio frequency energy is not input into any radio frequency circuit.

If the number of target radio frequency circuits of which the change amounts of the detection values reach the preset value range is less than the preset number, it does not meet requirement of the present radio frequency operation. Therefore, no radio frequency energy is output, that is, radio frequency energy is not input into any radio frequency circuit. It is also possible to simultaneously trigger an alarm module to issue an alarm, including flashing of an alarm light and buzzing.

In this embodiment of this application, when an operated object is connected to a radio frequency host through connecting ends of radio frequency circuits, according change amounts of detection values of the radio frequency circuits, it is determined whether a number of target radio frequency circuits of which the change amounts reach a preset value range reaches a preset number, that is, it is determined whether the connection between the connecting ends and the operated object meets a connection standard; if reaching, the preset number of target radio frequency circuits are selected from the target radio frequency circuits as radio frequency input circuits, and radio frequency signals are controlled to input; if not reaching, no radio frequency is input, so as to avoid subsequent radio frequency operations from being affected by connection not meeting the standard. Accordingly, the above-described method for safety control of radio frequency operation can automatically determine whether connection between an operated object and radio frequency circuits meets a standard, and does not input radio frequency energy when the number of radio frequency circuits meeting the standard do not meet requirement of radio frequency operation, thereby improving safety and intelligence of radio frequency operation.

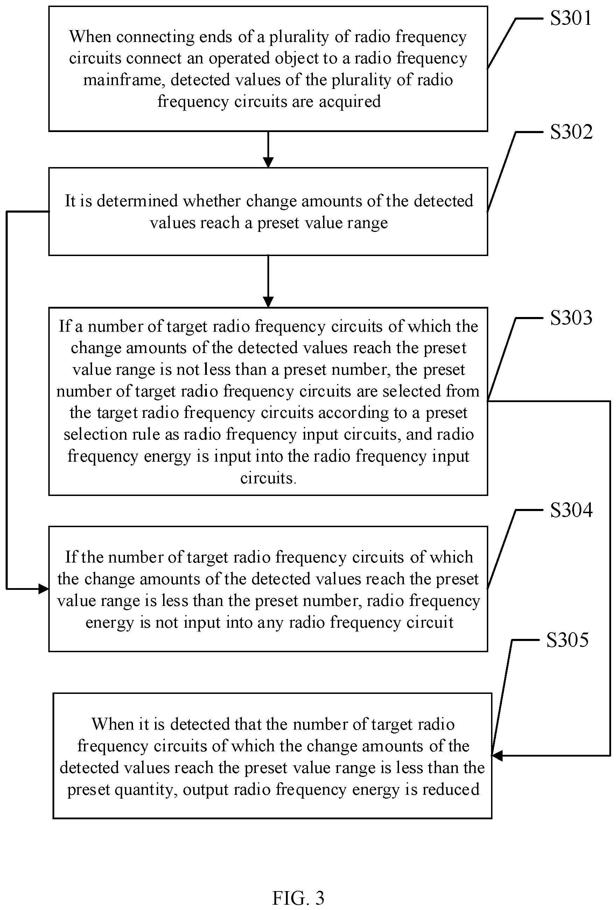

Referring to FIG. 3, which is an implementation flow chart of a method for safety control of radio frequency operation provided by another embodiment of this application. The method can be applied to the radio frequency host shown in FIG. 1, as shown in FIG. 3, the method specifically comprises the steps as follows.

Step S301, when connecting ends of a plurality of radio frequency circuits connect an operated object to a radio frequency host, detection values of the plurality of radio frequency circuits are acquired.

Step S302, it is determined whether change amounts of the detection values reach a preset value range.

Specifically, a change amount of a detection value is a change amount of a voltage of a primary coil of a transformer in a radio frequency circuit.

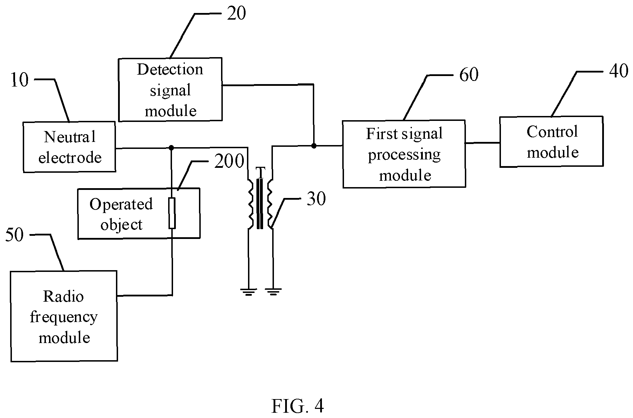

Referring to FIG. 4, FIG. 4 is a structural schematic diagram of a radio frequency circuit. Each radio frequency circuit has a connecting end, that is, a neutral electrode 10. In this embodiment, it is possible to connect a plurality of radio frequency circuits with an operated object at the same time. Furthermore, each radio frequency further includes a detection signal module 20, a transformer 30, a control module 40, and a radio frequency module 50; wherein the detection signal module 20 and the neutral electrode module 10 are respectively connected to a primary coil and a secondary coil of the transformer 30, and the neutral electrode 10 together with the secondary coil of the transformer 30 forms a loop when being attached to the operated object. The detection signal module 20 sends a detecting signal; the control module 40 can specifically be processor such as a CPU, which may be a CPU in the radio frequency host and may also be an individual CPU of the radio frequency circuit, and can control the detection signal module 20 to input the detecting signal into the circuit, and can also determine whether a connection between the neutral electrode 10 and the radio frequency host meets a connection standard according to a decrease value of a voltage of the primary coil of the transformer 30, and control the radio frequency module 50 to output radio frequency energy to the operated object 200. The radio frequency circuit further includes a first signal processing module 60, the first signal processing module 60 includes a filter, a resonator, an amplifier, etc., so as to perform filtering, amplification, and so on for the detection signal, and the specific circuit structure is not particularly limited.

Specifically, when neutral electrodes of a plurality of radio frequency circuits are attached to the operated object to connect the operated object to the radio frequency host, the control module controls the detection signal module to send a detecting signal to the transformer, and acquires voltage values of primary coils of transformers of the plurality of radio frequency circuits. When a voltage value decreases to the preset value range, it is determined that an attachment degree between a neutral electrode 10 and the operated object 200 meets a preset standard. The preset value range is preferably 1.6-2.0 V (volt).

A specific implementation manner in a circuit can be that: it is possible to set a high electric level signal and a low electric level signal according to the preset value range, for example, a voltage signal of 2.3-2.8V is set to be the high electric level signal, and a voltage signal of 0.6-1.0V is set to be the low electric level signal. Thus, when the control module detects that a detecting signal changes from the high electric level signal to the low electric level signal, it can be determined that an attachment degree between a neutral electrode 10 and the operated object 200 meets the preset standard; if a change amount of a voltage value does not reach the preset value range, the level of the signal does not generate an obvious change, the control module 40 determines that an attachment degree between a neutral electrode 10 and the operated object 200 does not meet the preset standard.

Step S303, when a number of target radio frequency circuits of which the change amounts of the detection values reach the preset value range is not less than a preset number, the preset number of target radio frequency circuits are selected from the target radio frequency circuits according to a preset selection rule as radio frequency input circuits, and radio frequency energy is input into the radio frequency input circuits.

Among them, the step of selecting the preset number of target radio frequency circuits from the target radio frequency circuits according to a preset selection rule as radio frequency input circuits specifically comprises: acquiring preset numbers of the target radio frequency circuits; determining connecting areas corresponding to the target radio frequency circuits according to the numbers; and correspondingly selecting the radio frequency input circuits in the target radio frequency circuits according to operation areas of the current radio frequency operation and a number of circuits required by each operation area.

Step S304, if the number of target radio frequency circuits of which the change amounts of the detection values reach the preset value range is less than the preset number, radio frequency energy is not input into any radio frequency circuit.

Technical details of the above steps refer to the descriptions of the embodiment shown in above FIG. 2, and are not repeated here.

Step S305, when it is detected that the number of target radio frequency circuits of which the change amounts of the detection values reach the preset value range is less than the preset number, the output radio frequency energy is reduced.

After radio frequency energy is input, if it is detected that the number of target radio frequency circuits of which the change amounts of the detection values reach the preset value range is less than the preset number, it means that there is a problem in the connection between a connecting end currently inputting radio frequency energy and the operated object, and a connection standard is not met. If the number of radio frequency circuits that meet the connection standard does not reach a preset number, that is, requirement of the radio frequency operation is not met, output radio frequency energy is reduced to avoid causing damage to the radio frequency host or the radio frequency operated object. At the same time, an alarm can be performed to prompt operating users to check the connection situation between the connection ends and the operated object. The alarm can be an audible and visual alarm or text display on a display screen of the radio frequency host.

Here, a method of detecting whether the change amounts of the detection values reaches the preset value range can be implemented by detecting a decreasing amount of a voltage of a primary coil of a transformer, as in the above step S302, and can also be implement by detecting an output impedance value of a radio frequency input circuit, which are specifically as follows.

Specifically, reducing output radio frequency energy means decreasing power of an output radio frequency signal. In this case, radio frequency energy is reduced to be a value that is not equal to 0, and radio frequency energy with a safe strength is still output. Alternatively, the connection between the radio frequency input circuit and the operated object is cut off; in this case, radio frequency energy is directly reduced to 0.

In this embodiment of the this application, when an operated object is connect to a radio frequency host through connecting ends of radio frequency circuits, before radio frequency energy is input, according change amounts of detection values of the radio frequency circuits, it is determined whether a number of target radio frequency circuits of which the change amounts reach a preset value range reaches a preset number, that is, it is determined whether the connection between the connecting ends and the operated object meets a connection standard; if reaching, the preset number of target radio frequency circuits are selected from the target radio frequency circuits as radio frequency input circuits, and radio frequency signals are controlled to input; if not reaching, no radio frequency is input, so as to avoid subsequent radio frequency operations from being affected by connection noting meet the standard. Accordingly, the above-described method for safety control of radio frequency operation can automatically determine whether connection between an operated object and radio frequency circuits meets a standard, and does not input radio frequency energy when the number of radio frequency circuits meeting the standard do not meet requirement of radio frequency operation, thereby improving safety and intelligence of radio frequency operation. Furthermore, after radio frequency energy is input, it is further possible to detect the change amounts of the detection values continuously and in real time; if it is detected that the number of target radio frequency circuits of which the change amounts of the detection values reach the preset value range is less than the preset number, output radio frequency energy is reduced, so as to reduce risk of damage to the radio frequency host and the operated object, and further improve safety and intelligence of radio frequency operations.

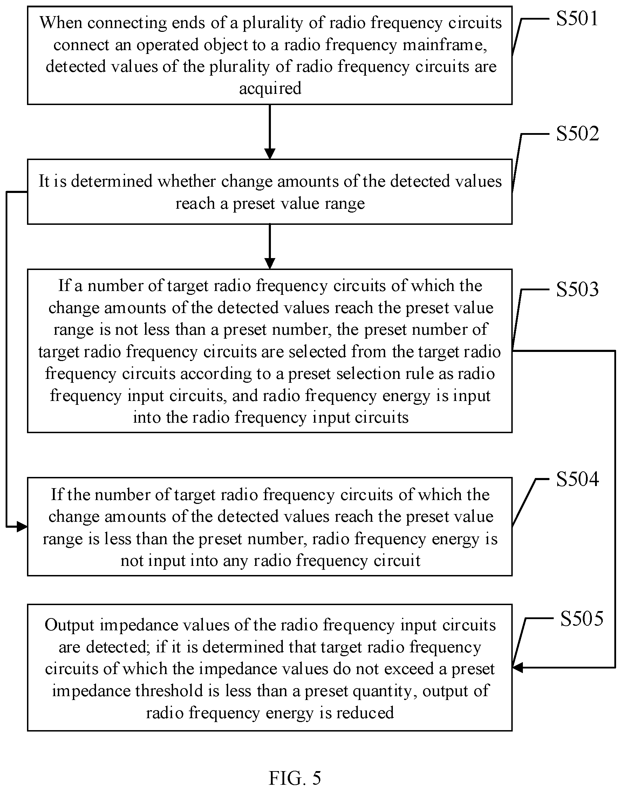

Referring to FIG. 5, which is an implementation flow chart of a method for safety control of radio frequency operation provided by another embodiment of this application. The method can be applied to the radio frequency host shown in FIG. 1, as shown in FIG. 5, the method specifically comprises the steps as follows.

Step S501, when connecting ends of a plurality of radio frequency circuits connects an operated object to a radio frequency host, detection values of the plurality of radio frequency circuits are acquired.

Step S502, it is determined whether change amounts of the detection values reach a preset value range.

Step S503, if a number of target radio frequency circuits of which the change amounts of the detection values reach the preset value range is not less than a preset number, the preset number of target radio frequency circuits are selected from the target radio frequency circuits according to a preset selection rule as radio frequency input circuits, and radio frequency energy is input into the radio frequency input circuits.

Step S504, if the number of target radio frequency circuits of which the change amounts of the detection values reach the preset value range is less than the preset number, radio frequency energy is not input into any radio frequency circuit.

Step S505, output impedance values of the radio frequency input circuits are detected; if it is determined that the number of target radio frequency circuits of which the impedance values do not exceed a preset impedance threshold is less than a preset number, output of radio frequency energy is reduced.

The output impedance value of each impedance detection circuit is detected, it is determined whether the impedance values exceed a preset impedance threshold, and the number of target radio frequency circuits of which the impedance values do not exceed the preset impedance threshold is counted. If the target radio frequency circuits of which the impedance values do not exceed the preset impedance threshold is less than the preset number, a frequency of an output radio frequency signal is reduced or a connection between a radio frequency input circuit and the operated object is cut off.

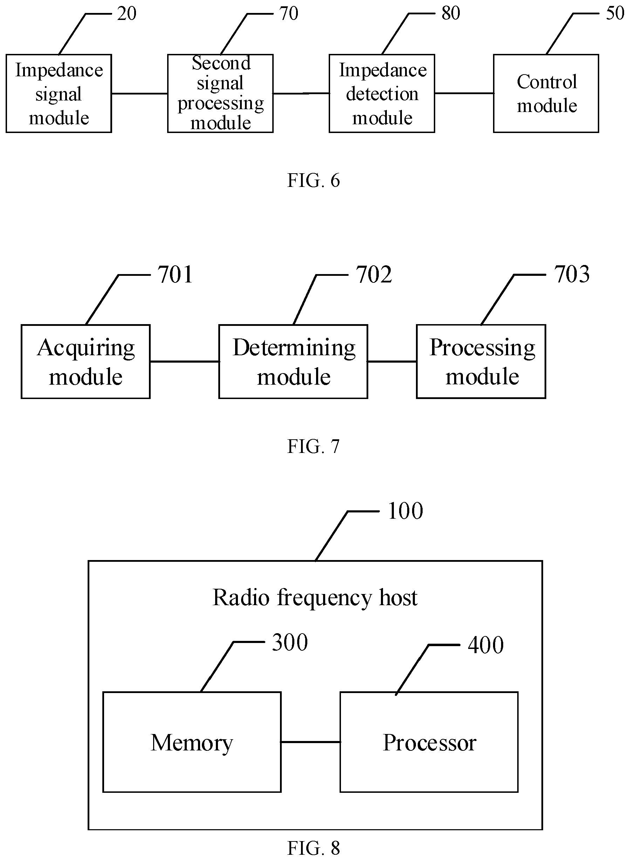

Referring to FIG. 6, FIG. 6 is a structural schematic diagram of an impedance detection circuit. Each impedance detection circuit is connected to an output end of a radio frequency input circuit to which a radio frequency signal is input, and each impedance detection circuit includes an impedance detection signal module 60, a second signal processing module 70, an impedance detection module 80, and the control module 40. The second signal processing module 70 includes a filter, a resonator, an amplifier, etc., so as to perform filtering, amplification, and so on for an impedance detection signal, and the specific circuit structure is not particularly limited. The impedance detection module 80 may include an impedance detection circuit configured to directly detect impedance values, or it may include a current detection circuit and a voltage detection circuit for indirectly calculating impedance values based on detected currents and voltages, the specific circuit structure is not particularly limited.

In this embodiment of the this application, when an operated object is connect to a radio frequency host through connecting ends of radio frequency circuits, before radio frequency energy is input, according change amounts of detection values of the radio frequency circuits, it is determined whether a number of target radio frequency circuits of which the change amounts reach a preset value range reaches a preset number, that is, it is determined whether the connection between the connecting ends and the operated object meets a connection standard; if reaching, the preset number of target radio frequency circuits are selected from the target radio frequency circuits as radio frequency input circuits, and radio frequency signals are controlled to input; if not reaching, no radio frequency is input, so as to avoid subsequent radio frequency operations from being affected by connection not meeting the standard. Accordingly, the above-described method for safety control of radio frequency operation can automatically determine whether connection between an operated object and radio frequency circuits meets a standard, and does not perform radio frequency energy input when the number of radio frequency circuits meeting the standard do not meet requirement of radio frequency operation, thereby improving safety and intelligence of radio frequency operation. Furthermore, after radio frequency energy is input, it is further possible to detect impedance values of the radio frequency input circuit in real time; if it is detected that the number of target radio frequency circuits of which the impedance values exceed a preset impedance threshold and reach a preset value range is less than a preset number, output radio frequency energy is reduced, so as to reduce risk of damage to the radio frequency host and the operated object, and further improve safety and intelligence of radio frequency operations.

Referring to FIG. 7, which is a structural schematic diagram of a device for safety control of radio frequency operation provided by an embodiment of this application. For convenience of descriptions, only parts related to embodiments of this application are shown. The device can be arranged in the above-described radio frequency main frame, and the device includes:
an acquiring module 701 configured to acquire detection values of a plurality of radio frequency circuits when connecting ends of the plurality of radio frequency circuits connect an operated object to a radio frequency host;
a determining module 702 configured to determine whether change amounts of the detection values reach a preset value range; and
a processing module 703 configured to: select a preset number of target radio frequency circuits from target radio frequency circuits according to a preset selection rule as radio frequency input circuits, and input radio frequency energy into the radio frequency input circuits when a number of the target radio frequency circuits of which the change amounts of the detection values reach the preset value range is not less than a preset number;
wherein the processing module 703 is further configured not to input radio frequency energy into any radio frequency circuit when the number of target radio frequency circuits of which the change amounts of the detection values reach the preset value range is less than the preset number.

Furthermore, the processing module 703 is further configured to: reduce output radio frequency energy, specifically for reducing power of an output radio frequency signal; or cut off a connection between a radio frequency input circuit and an operated object when it is detected that the number of target radio frequency circuits of which the change amounts of the detection values reach the preset value range is less than the preset number.

The processing module 703 is further configured to: acquire preset series numbers of the target radio frequency circuits; determine connecting areas corresponding to the target radio frequency circuits according to the series numbers; and correspondingly select the radio frequency input circuits in the target radio frequency circuits according to operation areas of the current radio frequency operation and a number of circuits required by each operation area.

Furthermore, the connecting end is a neutral electrode, each radio frequency circuit has a neutral electrode, and each radio frequency circuit includes a detection signal module, a transformer, and a control module.

Thus, the processing module 703 is further configured to: control the control module to control the detection signal module to send a detecting signal to the transformer, and acquire voltage values of primary coils of transformers of a plurality of radio frequency circuits when the neutral electrodes of the plurality of radio frequency circuits are attached to the operated object to connect the operated object to the radio frequency host.

Furthermore, the determining module 702 is further configured to determine whether the voltage values of primary coils of transformers of radio frequency circuits are decreased to reach a preset value range.

The processing module 703 is further configured to: detect an output impedance value of each radio frequency input circuit, determine whether the impedance values exceed a preset impedance threshold, and count a number of target radio frequency circuits of which the impedance values do not exceed the preset impedance threshold; and reduce a frequency of an output radio frequency signal or cut off a connection between a radio frequency input circuit and the operated object when the target radio frequency circuits of which the impedance values do not exceed the preset impedance threshold is less than the preset number,.

Specifically, the processing module 703 detects the output impedance values by controlling impedance detection circuits. Among them, each impedance detection circuit is connected to an output end of a radio frequency input circuit, and each impedance detection circuit includes an impedance detection signal module, an impedance detection module, and the control module. The processing module 703 controls the impedance detection signal module through the control module to output an impedance detection signal, and the impedance detection module detects an output impedance value of each radio frequency input circuit.

In this embodiment of the this application, when an operated object is connect to a radio frequency host through connecting ends of radio frequency circuits, before radio frequency energy is input, according to change amounts of detection values of the radio frequency circuits, it is determined whether a number of target radio frequency circuits of which the change amounts reach a preset value range reaches a preset number, that is, it is determined whether the connection between the connecting ends and the operated object meets a connection standard; if reaching, the preset number of target radio frequency circuits are selected from the target radio frequency circuits as radio frequency input circuits, and radio frequency signals are controlled to input; if not reaching, no radio frequency is input, so as to avoid subsequent radio frequency operations from being affected by connection not meeting the standard. Accordingly, the above-described method for safety control of radio frequency operation can automatically determine whether connection between an operated object and radio frequency circuits meets a standard, and does not input radio frequency energy when the number of radio frequency circuits meeting the standard do not meet requirement of radio frequency operation, thereby improving safety and intelligence of radio frequency operation. Furthermore, after radio frequency energy is input, it is further possible to detect the change amounts of the detection values or the detection values in real time; if it is detected that the change amounts of the detection values reach a preset value range, or the number of target radio frequency circuits of which the impedance values exceed a preset impedance threshold and reach a preset value range is less than a preset number, the output radio frequency energy is reduced, so as to reduce risk of damage to the radio frequency host and the operated object, and further improve safety and intelligence of radio frequency operations.

As shown in FIG. 8, an embodiment of this application further provides a radio frequency host, which includes a memory 300 and a processor 400, the processor 400 may be a device for safety control of radio frequency operation in the above embodiment, and may also be the processing module 703 in the device for safety control of radio frequency operation. The memory 300 may be, for example, a hard disk drive memory, a non-volatile memory (such as a flash memory or other electronically programmable restricted deletion memory used to form solid-state drives, etc.), volatile memory (such as a static or dynamic random access memory, etc.), and so on, embodiments of this application do not limit here.

The memory 300 stores executable program codes; the processor 400 coupled with the memory 300 calls the executable program codes stored in the memory to execute the above-described method for safety control of radio frequency operation.

Further, an embodiment of this application further provides a computer-readable storage medium, the computer-readable storage medium may be set in the radio frequency hosts in the above embodiments, and the computer-readable storage medium can be the memory 300 in the above embodiment shown in FIG. 8. The computer-readable storage medium stores a computer program, and the program, when being executed by a processor, implements the method for safety control of radio frequency operation described in the embodiments shown in above FIG. 2, FIG. 3, and FIG. 5. Further, the computer-readable storage medium may also be a U-disk, a mobile hard disk, a read-only memory (ROM), a RAM, a magnetic disk or a CD-ROM, and other media that can store program codes.

It should be noted that regarding the foregoing method embodiments, for simplicity of description, they are all expressed as a series of action combinations, but those skilled in the art should know that the present invention is not limited by the described sequence of actions. Because according to the present invention, certain steps can be performed in other orders or simultaneously. Secondly, those skilled in the art should also know that the embodiments described in the specification are all preferred embodiments, and the involved actions and modules are not necessarily all required by the present invention.

In the above-mentioned embodiments, the description of each embodiment has its own emphasis. For parts that are not described in detail in a certain embodiment, reference may be made to related descriptions of other embodiments.

The above is a description of the method and device for safety control of radio frequency operation, and the radio frequency host provided by the present invention. For those skilled in the art, according to the ideas of the embodiments of the present invention, both the specific implementation and the application scope will have changed parts. In summary, the content of this specification should not be construed as a limitation to the present invention.

## Claims

1. A method for safety control of radio frequency operation, comprising steps of:
when connecting ends of a plurality of radio frequency circuits connect an operated object to a radio frequency host, acquiring detection values of the plurality of radio frequency circuits;
determining whether change amounts of the detection values reach a preset value range;
when a number of target radio frequency circuits of which the change amounts of the detection values reach the preset value range is not less than a preset number, selecting the preset number of target radio frequency circuits from the target radio frequency circuits according to a preset selection rule as radio frequency input circuits, and inputting radio frequency energy into the radio frequency input circuits;
when the number of target radio frequency circuits of which the change amounts of the detection values reach the preset value range is less than the preset number, not inputting radio frequency energy to any radio frequency circuit.

2. The method according to claim 1, further comprising, after the step of inputting radio frequency energy into the radio frequency input circuits, a step of:
when detecting that the number of target radio frequency circuits of which the change amounts of the detection values reach the preset value range is less than the preset number, reducing output radio frequency energy.

3. The method according to claim 2, wherein the step of reducing output radio frequency energy comprises:
reducing power of an output radio frequency signal, or cutting off a connection between a radio frequency input circuit and the operated object.

4. The method according to any one of claims 1 to 3, wherein the step of selecting the preset number of target radio frequency circuits from the target radio frequency circuits according to a preset selection rule as radio frequency input circuits comprises steps of:
acquiring preset series numbers of the target radio frequency circuits; determining connecting areas corresponding to the target radio frequency circuits according to the series numbers; and
correspondingly selecting the radio frequency input circuits in the target radio frequency circuits according to operation areas of the current radio frequency operation and a number of circuits required by each operation area.

5. The method according to claim 4, wherein the connecting end is a neutral electrode, each radio frequency circuit has a neutral electrode, and each radio frequency circuit includes a detection signal module, a transformer, and a control module;
the step of when connecting ends of a plurality of radio frequency circuits connects an operated object to a radio frequency host, acquiring detection values of the plurality of radio frequency circuits comprises:
when neutral electrodes of a plurality of radio frequency circuits are attached to the operated object to connect the operated object to the radio frequency host, controlling, by the control module, the detection signal module to send a detecting signal to the transformer, and acquire voltage values of primary coils of transformers of the plurality of radio frequency circuits.

6. The method according to claim 5, wherein the step of determining whether change amounts of the detection values reach a preset value range comprises:
determining whether the voltage values of primary coils of transformers of radio frequency circuits are reduced to reach a preset value range.

7. The method according to claim 6, further comprising, after the step of inputting radio frequency energy into the radio frequency input circuits, steps of:
detecting an output impedance value of each radio frequency input circuit;
determining whether the impedance values exceed a preset impedance threshold, and counting a number of target radio frequency circuits of which the impedance values do not exceed the preset impedance threshold; and
when the target radio frequency circuits of which the impedance values do not exceed the preset impedance threshold is less than the preset number, reducing a frequency of an output radio frequency signal or cutting off a connection between a radio frequency input circuit and the operated object.

8. The method according to claim 7, wherein the step of detecting an output impedance value of each radio frequency input circuit comprising:
detecting the output impedance values by impedance detection circuits;
wherein each impedance detection circuit is connected to an output end of a radio frequency input circuit, and each impedance detection circuit includes an impedance detection signal module, an impedance detection module, and the control module; and
wherein the control module controls the impedance detection signal module to output an impedance detection signal, and the impedance detection module detects an output impedance value of each radio frequency input circuit.

9. A device for safety control of radio frequency operation, comprising:
an acquiring module configured to acquire detection values of a plurality of radio frequency circuits when connecting ends of the plurality of radio frequency circuits connect an operated object to a radio frequency host;
a determining module configured to determine whether change amounts of the detection values reach a preset value range; and
a processing module configured to: select s preset number of target radio frequency circuits from the target radio frequency circuits according to a preset selection rule as radio frequency input circuits, and input radio frequency energy into the radio frequency input circuits when a number of target radio frequency circuits of which the change amounts of the detection values reach the preset value range is not less than the preset number;
wherein the processing module is further configured not to input radio frequency energy into any radio frequency circuit when the number of target radio frequency circuits of which the change amounts of the detection values reach the preset value range is less than the preset number.

10. A radio frequency host, comprising:
a memory and a processor;
wherein the memory stores executable program codes;
the processor coupled with the memory calls the executable program codes stored in the memory to execute the method for safety control of radio frequency operation according to any one of claims 1 to 8.
